# EUROPEAN PATENT APPLICATION

(11) **EP 4 129 201 A1**
(43) Date of publication of application: **08.02.2023**
(21) Application number: 20926968.7
(22) Date of filing: 24.06.2020
(51) Int. Cl.: A61B 10/02

(54) **BIOPSY DEVICE, AND FIRING AND UNLOCKING MECHANISM, SAMPLING STROKE ADJUSTING MECHANISM, AND DRAWING AND FIRING METHOD THEREOF**

(30) Priority: 27.03.2020 CN 202010229277; 21.05.2020 CN 202010438242; 21.05.2020 CN 202010438241; 21.05.2020 CN 202010437616
(71) Applicant: Promisemed Hangzhou Meditech Co., Ltd., Hangzhou, Zhejiang 311100 (CN)
(72) Inventor: HU, Chaoyu, Hangzhou, Zhejiang 311100 (CN); GAO, Zhanqiang, Hangzhou, Zhejiang 311100 (CN)
(74) Representative: Cabinet Chaillot
(86) International application number: PCT/CN2020/097977
(87) International publication number: WO 2021/189688

(57) **Abstract**

Disclosed are a biopsy device, a cocking-unlocking mechanism, a sampling length adjusting mechanism and a spring-loading cocking method. A stylet (1), a needle tube (2) and an operating handle are included. The operating handle includes an installation frame, and the installation frame is internally provided with a first sliding block (6), a first cocking spring (7), a second sliding block (10) and a second cocking spring (11), and further provided with a spring-loading cocking mechanism for driving the first sliding block (6) and the second sliding block (10) to be moved backwards, locked and positioned and compressing the first cocking spring (7) and the second cocking spring (11) for potential storage, and a cocking-unlocking mechanism for releasing locking and positioning of the first sliding block (6) and/or the second sliding block (10). The biopsy device is reasonable in structure, convenient and comfortable to operate and has certain cost advantages.

## Description

### BACKGROUND

### Technical Field

The present disclosure relates to the field of medical equipment and a biopsy device for living tissue sampling, in particular to a multifunctional full-automatic biopsy device, a cocking-unlocking mechanism thereof, a sampling length adjusting mechanism and a spring-loading cocking method.

### Related Art

A biopsy device belongs to medical equipment, is applicable to living tissue sampling, cell aspiration, etc., for multiple organs such as livers, kidneys, lungs, prostate glands, mammary glands and thyroid glands, and has the characteristics of being simple and rapid in operation, convenient to use, rapid in sampling, and the like.

American Bard Company dominates the market due to its earlier research, development and sell of biopsy needle type products, and Japan TSK Company launches a full-automatic biopsy needle product following Bard, which brings more selections to doctors in clinic. A full-automatic biopsy needle type can achieve single-handed operation, is low in spring-loading difficulty, has a front cocking trigger and a rear cocking trigger with a shake-hand grip mode/a pen-hold grip mode facilitating the doctors to select according to practical situations, is simple and rapid in operation, rapid in sampling and low in risk, which is popular with many clinical doctors.

The patent literature with the publication number being CN104797200 discloses a biopsy device including a cutting intubation tube mechanism with a cutting intubation tube and an interior stylet mechanism with an interior stylet, the interior stylet is coaxial to the cutting intubation tube. A cocking mechanism is configured to cock the cutting intubation tube mechanism and the interior stylet mechanism by enabling the cutting intubation tube and the interior stylet to return to a cocking position in a proximal-side direction. An actuator device is configured to launch at least one of the interior stylet mechanism and the cutting intubation tube mechanism so that at least one of the interior stylet and the cutting intubation tube can correspondingly advance from the cocking position in a distal-side direction. A selector assembly includes a selector switch with an exterior lug capable of being touched by a user. The selector assembly is configured for selection between at least two operation modes selected by the user and at least two launching distances selected by the user.

But, biopsy devices sold on the market at present have certain defects, for example, a cocking device is not high in reliability and may have an unable cocking or difficult cocking situation; a safety button is not designed for the cocking device, which easily causes a false cocking situation; since an inner needle and an outer needle are cocked in linkage, a sampling effect fed back by customers is not ideal; and in addition, a full-automatic needle needs to be spring-loaded through outward pulling by two hands rather than one hand, which causes complex operation; and a product only has one cocking position/cocking button which cannot be transversely gripped, and consequently, usage is inconvenient. In addition, there is generally only one sampling length (full-length sampling or whole-channel sampling), and when only a few tissues need to be sampled, a biopsy device with a shorter sampling length needs to be adopted through replacement, which causes inconvenient usage and increased usage costs.

### SUMMARY

To solve above technical problems, the present disclosure aims to provide a biopsy device, a cocking-unlocking mechanism thereof, a sampling length adjusting mechanism and a spring-loading cocking method, which are reasonable in structure, convenient and comfortable to operate and have certain cost advantages.

In order to achieve the above purposes, the present disclosure adopts a following technical solution:
a biopsy device includes a stylet, a needle tube and an operating handle for controlling the stylet and the needle tube to perform biopsy sampling; the operating handle includes an installation frame internally provided with a first sliding block, a first cocking spring, a second sliding block and a second cocking spring; a rear end of the needle tube is fixedly connected to the first sliding block, a rear end of the stylet penetrates out of the needle tube and the first sliding block and then is fixedly connected to the second sliding block, a front end of the first cocking spring abuts against the first sliding block, a rear end of the first cocking spring abuts against the installation frame, a front end of the second cocking spring abuts against the second sliding block, and a rear end of the second cocking spring abuts against the installation frame; and the installation frame is further provided with a spring-loading cocking mechanism for driving the first sliding block and the second sliding block to be moved backwards, locked and positioned and compressing the first cocking spring and the second cocking spring for potential storage, and the cocking-unlocking mechanism for releasing locking and positioning of the first sliding block and/or the second sliding block.

Preferably, the cocking-unlocking mechanism includes a first spring-loading button and a second spring-loading button, the first spring-loading button is connected to the first sliding block and can drive the first sliding block to move backwards, the first sliding block is provided with a first elastic barb, after moving backwards in place, the first sliding block is hooked and locked, by the first elastic barb with a first locking portion arranged on the installation frame so as to achieve spring-loading, the second spring-loading button is connected to the second sliding block and can drive the second sliding block to move backwards, the second sliding block is provided with a second elastic barb, and after moving backwards in place, the second sliding block is hooked and locked, by the second elastic barb with a second locking portion arranged on the installation frame so as to achieve spring-loading.

Preferably, a front portion of the installation frame is provided with a first slideway, the first sliding block and the first cocking spring are arranged in the first slideway, a rear portion of the installation frame is provided with a second slideway, the second sliding block and the second cocking spring are arranged in the second slideway, barrier portions are arranged at the front end of the first slideway, the rear end of the second slideway and between the first slideway and the second slideway, accordingly, the first sliding block is limited to slide in the first slideway, and the second sliding block is limited to slide in the second slideway; the installation frame is provided with the first locking portion and a second locking portion, the first locking portion is a convex lug, a front side of the first locking portion is provided with a slope surface for promoting the first elastic barb on the first sliding block to be elastically contracted and deformed, a rear side of the first locking portion is a clamping surface capable of being clamped and locked to a free end of the first elastic barb, the second locking portion is a convex lug, a front side of the second locking portion is provided with a slope surface for promoting the second elastic barb on the second sliding block to be elastically contracted and deformed, and a rear side of the second locking portion is a clamping surface capable of being clamped and locked to a free end of the second elastic barb; a handle shell is fixed to the exterior of the installation frame, the first spring-loading button and the second spring-loading button are arranged at a front end of the handle shell side by side from left to right, an extension arm, stretching into the handle shell, of the first spring-loading button is connected to the first sliding block so as to backwards push the first sliding block, an extension arm, stretching into the handle shell, of the second spring-loading button is connected to the second sliding block so as to backwards push the second sliding block, and the extension arm of the first spring-loading button and the extension arm of the second spring-loading button are arranged on a left side and a right side of the installation frame respectively.

Preferably, a first damping gasket is arranged at a front end of the first sliding block, and a second damping gasket is arranged at a front end of second sliding block.

Preferably, a cocking connecting rod is pushed by a rear cocking button or a side cocking button exposed out of the handle shell, and the rear cocking button or the side cocking button and the cocking connecting rod are integrally formed or fixedly matched.

A cocking-unlocking mechanism of a biopsy device includes an installation frame, a first sliding block, a first cocking spring, a second sliding block, a second cocking spring and a cocking connecting rod are arranged on the installation frame, the first sliding block is fixedly connected to a rear end of a needle tube, a front end of the first cocking spring abuts against the first sliding block, a rear end of the first cocking spring abuts against the installation frame, the first sliding block is provided with a first elastic barb, and after moving backwards in place, the first sliding block is hooked and locked, by the first elastic barb with a first locking portion arranged on the installation frame so as to achieve spring-loading; the second sliding block is fixedly connected to a rear end of a stylet, a front end of the second cocking spring abuts against the second sliding block, a rear end of the second cocking spring abuts against the installation frame, the second sliding block is provided with a second elastic barb, and after moving backwards in place, the second sliding block is hooked and locked, by the second elastic barb with a second locking portion arranged on the installation frame so as to achieve spring-loading; and where, the first sliding block and the second sliding block are respectively in front-back sliding connection to the installation frame and are arranged front and back; the cocking connecting rod is arranged on the installation frame, can move front and back, the cocking connecting rod is provided with a first cocking portion and a second cocking portion, the cocking connecting rod promotes, by the first cocking portion, the first elastic barb to be elastically deformed and separated from the first locking portion so as to unlock the first sliding block, and the cocking connecting rod promotes, by the second cocking portion, the second elastic barb to be elastically deformed and separated from the second locking portion, thereby unlocking the second sliding block. Accordingly, the elastic barbs are locked with the locking portions on the installation frame for spring-loading, the cocking connecting rod is irrelevant to spring-loading and locking, which makes locking stable and reliable, and cocking smooth and more labor-saving. The cocking connecting rod pushes, by the two cocking portions, the two elastic barbs to be elastically deformed to release locking and cocking, which achieves linked sequential cocking of the two sliding blocks, facilitates adjustment and design of sequential cocking interval time of the stylet and the needle tube, and sufficiently guarantees a desirable sampling effect, the structure is simple, usage is convenient, and running is reliable.

Preferably, the first locking portion is a convex lug, a front side of the first locking portion is provided with a slope surface for promoting the first elastic barb on the first sliding block to be elastically contracted and deformed, a rear side of the first locking portion is a clamping surface capable of being clamped and locked to a free end of the first elastic barb, the second locking portion is a convex lug, a front side of the second locking portion is provided with a slope surface for promoting the second elastic barb on the second sliding block to be elastically contracted and deformed, and a rear side of the second locking portion is a clamping surface capable of being clamped and locked to a free end of the second elastic barb.

Preferably, the cocking connecting rod is provided with the first cocking portion and the second cocking portion which are both in a slope shape, and correspondingly, the first elastic barb and the second elastic barb are provided with slopes matched with the first cocking portion and the second cocking portion respectively; and when the cocking connecting rod moves front and back for cocking, the second cocking portion firstly thrusts the second elastic barb on the second sliding block to be elastically contracted and deformed so as to be separated from the second locking portion, accordingly, the second sliding block connected to the stylet is unlocked and cocked, then, the first cocking portion thrusts the first elastic barb on the first sliding block to be elastically contracted and deformed so as to be separated from the first locking portion, and accordingly, the first sliding block connected to the needle tube is unlocked and cocked.

Preferably, the cocking connecting rod is provided with a reset portion, and a reset spring is arranged between the reset portion and the installation frame; and the installation frame is provided with a reset spring space and a fifth slideway, the reset spring is limited within the reset spring space, a rear end of the reset spring space communicates with the fifth slideway, the reset portion on the cocking connecting rod is inserted in the fifth slideway and may move front and back, and when the cocking connecting rod moves forwards, a front end of the reset spring abuts against the installation frame, and a rear end of the reset spring abuts against the reset portion on the cocking connecting rod.

A biopsy device includes the cocking-unlocking mechanism of the biopsy device.

A sampling length adjusting mechanism of the biopsy device includes an installation frame, a first sliding block, a first cocking spring, a second sliding block and a second cocking spring are arranged on the installation frame, the first sliding block is fixedly connected to a rear end of a needle tube, a front end of the first cocking spring abuts against the first sliding block, a rear end of the first cocking spring abuts against the installation frame, the first sliding block is provided with a first elastic barb, and after moving backwards in place, the first sliding block is hooked and locked, by the first elastic barb with a first locking portion arranged on the installation frame so as to achieve spring-loading; the second sliding block is fixedly connected to a rear end of a stylet, a front end of the second cocking spring abuts against the second sliding block, a rear end of the second cocking spring abuts against the installation frame, the second sliding block is provided with a second elastic barb, and after moving backwards in place, the second sliding block is hooked and locked, by the second elastic barb with a second locking portion arranged on the installation frame so as to achieve spring-loading; where the first sliding block and the second sliding block are respectively in front-back sliding connection to the installation frame and are arranged front and back; where and the sampling length adjusting mechanism further includes a cocking connecting rod for releasing unlocking and positioning of the first sliding block and/or the second sliding block, and a sampling length adjusting component, the sampling length adjusting component is provided with a hook boss, the sampling length adjusting component can move and at least has a locking state and an unlocking state, when the sampling length adjusting component is in the locking state, the hook boss is arranged on a front-back movement path of the first elastic barb on the first sliding block, and the first elastic barb can be hooked with the hook boss for positioning so as to achieve spring-loaded locking, and when the first sliding block is locked at the position, only part of a sampling channel of the stylet can be exposed out of the needle tube so as to implement incomplete-length sampling; and when the sampling length adjusting component is in the unlocking state, the hook boss leaves the front-back movement path of the first elastic barb on the first sliding block, and the first elastic barb can freely pass without obstruction. Accordingly, a spring-loading and locking position of the first sliding block can be selected by adjusting a pressing length of a first spring-loading button, thereby adjusting the biopsy device sampling length without manual operation of sampling length buttons for setting in advance, the structure is simple and reasonable, and operation is convenient and reliable.

Preferably, the cocking connecting rod is provided with the first cocking portion and the second cocking portion which are both in a slope shape, and correspondingly, the first elastic barb and the second elastic barb are provided with slopes matched with the first cocking portion and the second cocking portion respectively; and when the cocking connecting rod moves front and back for cocking, the second cocking portion firstly thrusts the second elastic barb on the second sliding block to be elastically contracted and deformed so as to be separated from the second locking portion, accordingly, the second sliding block connected to the stylet is unlocked and cocked, then, the first cocking portion thrusts the first elastic barb on the first sliding block to be elastically contracted and deformed so as to be separated from the first locking portion, and accordingly, the first sliding block connected to the needle tube is unlocked and cocked.

Preferably, the sampling length adjusting component is hinged to the installation frame, and can swing up and down, is provided with the hook boss and a release portion, an elastic member makes the sampling length adjusting component rotate and makes the hook boss get onto the front-back movement path of the first elastic barb on the first sliding block; the first elastic barb of the first sliding block moves backwards to be hooked with the hook boss for positioning so as to achieve spring-loaded locking, and when the first sliding block is locked at the position, only part of the sampling channel of the stylet can be exposed out of the needle tube; and the front end of the cocking connecting rod is provided with a thrust portion, when the cocking connecting rod moves forwards, the thrust portion abuts against the release portion and can promote the sampling length adjusting component to rotate so as to enable the hook boss to retreat from the front-back movement path of the first elastic barb.

Preferably, the thrust portion is located on one side of the first cocking portion, a contact surface, making contact with the release portion, of the thrust portion is a slope or a cambered surface, and a contact surface, making contact with the thrust portion, of the release portion is a slope or a cambered surface.

Preferably, the thrust portion and the first locking portion are located on a left side and a right side of the first cocking portion respectively, the thrust portion is located on a left side or a right side of the front-back movement path of the first elastic barb, and a width of a free end of the first elastic barb is larger than that of the thrust portion.

Preferably, the sampling length adjusting component is in a rod shape extending front and back, a hinge portion is arranged on a left side and a right side of the sampling length adjusting component, and the hinge portion is inserted in a hinge hole on the installation frame so that the sampling length adjusting component can be hinged to the installation frame; and the elastic member is a torsion spring sleeving the hinge portion, one end of the torsion spring abuts against the installation frame, the other end of the torsion spring abuts against a front end of the sampling length adjusting component, and the release portion is located at a rear end of the sampling length adjusting component.

The biopsy device includes the sampling length adjusting mechanism of the biopsy device. The installation frame is further provided with a spring-loading cocking mechanism for driving the first sliding block and the second sliding block to be moved backwards, locked and positioned and compressing the first cocking spring and the second cocking spring for potential storage; and the spring-loading cocking mechanism includes the first spring-loading button and a second spring-loading button, the first spring-loading button is connected to the first sliding block and can drive the first sliding block to move backwards, and the second spring-loading button is connected to the second sliding block and can drive the second sliding block to move backwards.

A spring-loading cocking method for full-length sampling by the biopsy device includes the following steps:
1) spring-loading,
   where, the first spring-loading button and the second spring-loading button are respectively pressed; the first sliding block is driven by the first spring-loading button to move backwards and compress the first cocking spring until the first elastic barb on the first sliding block is locked, and the first sliding block and the needle tube finish spring-loading; the second sliding block is driven by the second spring-loading button to move backwards and compress the second cocking spring until the second elastic barb on the second sliding block is locked, and the second sliding block and the stylet finish spring-loading; and
2) cocking,
where, the rear cocking button is pressed or the side cocking button is forwards pushed to drive the cocking connecting rod to move forwards; firstly, the cocking connecting rod promotes, by the second cocking portion, the second elastic barb to be elastically deformed and separated from the second locking portion so as to unlock the second sliding block, and the second sliding block is pushed by the second cocking spring to move forwards so as to finish unlocking and cocking of the second sliding block and the stylet; at the same time, the first cocking portion of the cocking connecting rod does not make contact with the first elastic barb on the first sliding block to maintain the locking state of the first sliding block; accordingly, the sampling channel of the stylet is exposed out of the front end of the needle tube; then, along with continuous forward movement of the cocking connecting rod, the cocking connecting rod promotes, by the first cocking portion, the first elastic barb to be elastically deformed and separated from the first locking portion so as to unlock the first sliding block, the first sliding block is pushed by the first cocking spring to move forwards so as to finish unlocking and cocking of the first sliding block and the needle tube, and the needle tube moves forwards so as to accommodate the sampling channel of the stylet in the front end; at the same time, the thrust portion of the cocking connecting rod abuts against the release portion and promotes the sampling length adjusting component to rotate so as to make the hook boss retreat from the front-back movement path of the first elastic barb, thereby avoiding contact between the first elastic barb and the hook boss in a cocking forward movement process; and finally, the rear cocking button and the side cocking button are loosened, the cocking connecting rod is pushed by the reset spring to move backwards and reset, and the sampling length adjusting component resets under the action of the elastic member.

A spring-loading cocking method for incomplete-length sampling by the biopsy device includes the following steps:
1) spring-loading,
   where, the first spring-loading button and the second spring-loading button are respectively pressed; the first sliding block is driven by the first spring-loading button to move backwards and compress the first cocking spring until the first elastic barb on the first sliding block is locked with the hook boss on the sampling length adjusting component, and the first sliding block and the needle tube finish spring-loading; and the second sliding block is driven by the second spring-loading button to move backwards and compress the second cocking spring until the second elastic barb on the second sliding block is locked, and the second sliding block and the stylet finish spring-loading; and
2) cocking,
where, the rear cocking button is pressed or the side cocking button is forwards pushed to drive the cocking connecting rod to move forwards; firstly, the cocking connecting rod promotes, by the second cocking portion, the second elastic barb to be elastically deformed and separated from the second locking portion so as to unlock the second sliding block, and the second sliding block is pushed by the second cocking spring to move forwards so as to finish unlocking and cocking of the second sliding block and the stylet; at the same time, the first cocking portion and the thrust portion of the cocking connecting rod do not make contact with the first elastic barb on the first sliding block to maintain the locking state of the first sliding block; accordingly, a part of the sampling channel of the stylet is exposed out of the front end of the needle tube; then, along with continuous forward movement of the cocking connecting rod, the thrust portion at the front end of the cocking connecting rod abuts against the release portion and can promote the sampling length adjusting component to rotate so as to make the hook boss retreat from the front-back movement path of the first elastic barb, thereby releasing clamping and locking, by the hook boss, on the first elastic barb, the first sliding block is pushed by the first cocking spring to move forwards so as to finish unlocking and cocking of the first sliding block and the needle tube, and the needle tube moves forwards to accommodate the sampling channel of the stylet in the front end; and finally, the rear cocking button and the side cocking button are loosened, the cocking connecting rod is pushed by the reset spring to move backwards and reset, and the sampling length adjusting component resets under the action of the elastic member.

Due to adoption of the above technical solution in the present disclosure, the stylet and the needle tube can be sequentially cocked, the tissue can sufficiently fall into the internal needle sampling channel, the sampling effect is good, the structure is reasonable, operation is convenient and comfortable, and certain cost advantages are achieved. The present disclosure adopts the safety button, which can effectively prevent misoperation and make a sampling process safer. The present disclosure sets the adjustable sampling length, which can be applicable to pathological tissues different in size, obtain tissue specimens different in length, has an advantage of wide in application, and can reduce hurt to healthy tissues and effectively reduce pains of patients. In addition, the sliding blocks are provided with the damping gaskets capable of buffering impact force from the sliding blocks and reduce a sense of fear brought to the patients by work noise, and usage experience of users is good.

### BRIEF DESCRIPTION OF THE DRAWINGS

FIG. 1 is a perspective view of an Embodiment 1;
FIG. 2 is an explosive view of the Embodiment 1;
FIG. 3 is a structural schematic diagram of a cocking connecting rod;
FIG. 4 is a structural schematic diagram of cooperation of the cocking connecting rod and a sampling length adjusting component;
FIG. 5 is a structural schematic diagram of cooperation of a first installation frame and related components;
FIG. 6 is a structural schematic diagram of cooperation of the first installation frame, the cocking connecting rod and related components;
FIG. 7 is a structural schematic diagram (first view angle) of cooperation of two installation frames, the cocking connecting rod and related components;
FIG. 8 is a structural schematic diagram of cooperation of a second installation frame, the cocking connecting rod and related components;
FIG. 9 is a structural schematic diagram (second view angle) of cooperation of the two installation frames, the cocking connecting rod and related components;
FIG. 10 is a broken-out section view (safety button locking) of the Embodiment 1;
FIG. 11 is a first spring-loading schematic diagram of the Embodiment 1;
FIG. 12 is a second spring-loading schematic diagram of the Embodiment 1;
FIG. 13 is a broken-out section view (safety button opening for full-length sampling) in a spring-loading state of the Embodiment 1;
FIG. 14 is a first cocking schematic diagram of the Embodiment 1;
FIG. 15 is a second cocking schematic diagram of the Embodiment 1;
FIG. 16 is a structural schematic diagram (the cocking connecting rod resets) in a cocking state of the Embodiment 1;
FIG. 17 is a first spring-loading schematic diagram of incomplete-length sampling of the Embodiment 1;
FIG. 18 is a second spring-loading schematic diagram of incomplete-length sampling of the Embodiment 1;
FIG. 19 is a cocking schematic diagram of incomplete-length sampling of the Embodiment 1;
FIG. 20(a) is a first full-length sampling schematic diagram;
FIG. 20(b) is a second full-length sampling schematic diagram;
FIG. 20(c) is a third full-length sampling schematic diagram;
FIG. 21(a) is a first incomplete-length (half-length) sampling schematic diagram;
FIG. 21(b) is a second incomplete-length (half-length) sampling schematic diagram; and
FIG. 21(c) is a third incomplete-length (half-length) sampling schematic diagram.

Where, 1-stylet, 2-needle tube, 3-cannula, 4-first shell, 5-first damping gasket, 6-first sliding block, 61-first elastic barb, 62-first connecting portion, 7-first cocking spring, 8-first installation frame, 81-first slideway, 82-first slot hole, 83-second slideway, 84-second slot hole, 85-first locking portion, 86-fifth slideway, 87-reset spring space, 88-second locking portion, 89-hinge hole, 9-second damping gasket, 10-second sliding block, 101-second elastic barb, 102-second connecting portion, 11-second cocking spring, 12-safety button, 13-cocking connecting rod, 131-rear cocking button, 132-locking groove, 133-first cocking portion, 134-reset portion, 135-connecting portion, 136-second cocking portion, 137-thrust portion, 14-first spring-loading button, 15-sampling length adjusting component, 151-hook boss, 152-release portion, 153-hinge portion, 16-elastic member, 17-side cocking button, 171-limiting clamp lug, 18-reset spring, 19-second spring-loading button, 20-second shell, 22-second installation frame, and 220-positioning column.

### DETAILED DESCRIPTION

Embodiments of the present disclosure are described in detail below and shown in accompanying drawings, and mark numbers being the same or similar from beginning to end show same or similar members or members with same or similar functions. The following embodiments described in reference to the accompanying drawings are exemplary, and aim to explain the present disclosure instead of limiting the present disclosure.

In the description of the present disclosure, it needs to be understood that orientation or position relationships indicated by terms "center", "longitudinal", "transverse", "length", "width", "thickness", "upper", "lower", "front", "rear", "left", "right", "vertical", "horizontal", "top", "bottom", "inner", "clockwise", "anticlockwise", etc., are orientation or position relationships shown based on the accompanying drawings, are adopted not to indicate or imply that indicated devices or members must be in specific orientations or structured and operated in specific orientations but only to conveniently describe the present disclosure and simplify the description, and thus should not be understood as limits to the present disclosure.

In addition, terms "first" and "second" are only used for describing the purpose not understood as indicating or implying relative importance or implying to indicate the number of indicated technical features. Thus, features limited with "first" and "second" may indicate or invisibly include one or more features. In the description of the present disclosure, "a plurality of" means two or more unless otherwise noted or additional definite limitations.

In the present disclosure, except additional definite regulations and limitations, terms "install", "link", "connect", "fix", etc., should be generally understood, such as fixed connection, or detachable connection or integrated connection; or mechanical connection or electric connection; or direct connection, or indirect connection through intermediate media, or communication in two members. Ordinary skill in the art can understand specific meaning of the above terms in the present disclosure according to the specific situation.

In the present disclosure, except additional definite regulations and limitations, a first feature being "above" or "below" a second feature may include direct contact of the first feature and the second feature, and may include indirect contact of the first feature and the second feature through an additional feature therebetween. In addition, the first feature being "over", "above" and "on" the second feature includes the first feature being over and above the second feature, or merely indicates that a level of the first feature is higher than that of the second feature. The first feature being "under", "below" and "underneath" the second feature includes the first feature being under and below the second feature, or merely indicates that a level of the first feature is lower than that of the second feature.

### Embodiment 1:

To facilitate presentation and understanding, in the embodiment, a stylet sampling end indicates front, a handle end indicates back, and directions perpendicular to a front-back direction are respectively defined as a left-right direction and an up-down direction.

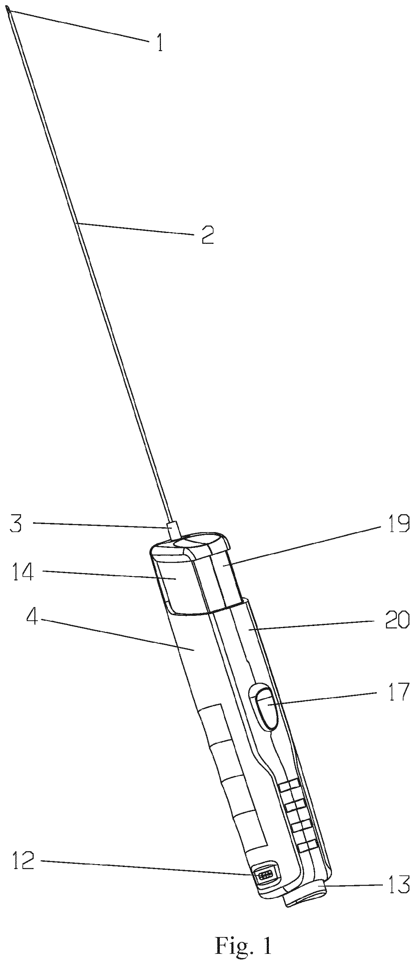

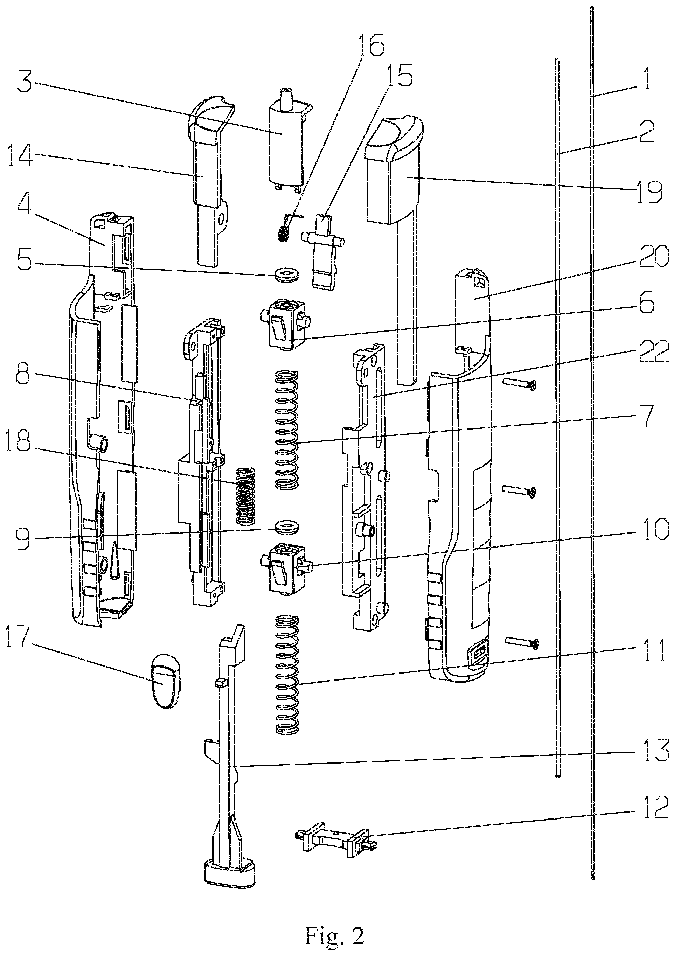

A biopsy device shown in FIG.1 and FIG.2 includes a stylet 1, a needle tube 2 and an operating handle for controlling the stylet and the needle tube to perform biopsy sampling. The operating handle includes an installation frame internally provided with a first sliding block 6, a first cocking spring 7, a second sliding block 10 and a second cocking spring 11. A rear end of the needle tube 2 is fixedly connected to the first sliding block 6, a rear end of the stylet 1 penetrates out of the needle tube 2 and the first sliding block 6 and then is fixedly connected to the second sliding block 10, a front end of the first cocking spring 7 abuts against the first sliding block 6, a rear end of the first cocking spring 7 abuts against the installation frame, a front end of the second cocking spring 11 abuts against the second sliding block 10, and a rear end of the second cocking spring 11 abuts against the installation frame; and the installation frame is further provided with a spring-loading cocking mechanism for driving the first sliding block 6 and the second sliding block 10 to be moved backwards, locked and positioned and compressing the first cocking spring 7 and the second cocking spring 11 for potential storage, and a cocking-unlocking mechanism for releasing locking and positioning of the first sliding block 6 and/or the second sliding block 10.

The spring-loading cocking mechanism includes a first spring-loading button 14 and a second spring-loading button 19, the first spring-loading button 14 is connected to the first sliding block 6 and may drive the first sliding block 6 to move backwards, the first sliding block 6 is provided with a first elastic barb 61, after moving backwards in place, the first sliding block 6 is hooked and locked, by the first elastic barb 61 with a first locking portion 85 arranged on the installation frame so as to achieve spring-loading, the second spring-loading button 19 is connected to the second sliding block 10 and may drive the second sliding block 10 to move backwards, the second sliding block 10 is provided with a second elastic barb 101, and after moving backwards in place, the second sliding block 10 is hooked and locked, by the second elastic barb 101 with a second locking portion 88 arranged on the installation frame so as to achieve spring-loading.

The cocking-unlocking mechanism includes a cocking connecting rod 13, the cocking connecting rod 13 is arranged on the installation frame, can move front and back, the cocking connecting rod 13 is provided with a first cocking portion 133 and a second cocking portion 136, the cocking connecting rod 13 promotes, by the first cocking portion 133, the first elastic barb 61 to be elastically deformed and separated from the first locking portion 85 so as to unlock the first sliding block 6, and the cocking connecting rod 13 promotes, by the second cocking portion 136, the second elastic barb 101 to be elastically deformed and separated from the second locking portion 88 so as to unlock the second sliding block 10.

In the embodiment, as shown in FIG.2, the installation frame includes a first installation frame 8 and a second installation frame 22 which are fixed in a left-right fit manner, a handle shell is fixed to the exterior of the installation frame and includes a first shell 4 and a second shell 20 which are fixed in a left-right fit manner, the first shell 4 and the second shell 20 are fixedly connected through a fastener or buckle, the first installation frame 8 and the second installation frame 22 are fixedly connected through fasteners, and the installation frame and the shell are fixed through cooperation of positioning columns 220 and positioning grooves. The structure is simple and reasonable, convenient to assemble and high in cost performance.

In the embodiment, the stylet 1 (such as the rear end of the stylet) has an anti-skid and anti-disengaging feature (such as concave and convex), and the stylet and the sliding block 10 may be fixedly and firmly connected (such as bond) due to the anti-skid feature; and the needle tube 2 (such as the rear end of the needle tube) has an anti-skid and anti-disengaging feature (such as a horn mouth and a flange face), and the needle tube and the sliding block 6 may be firmly connected (such as bond) due to the anti-skid feature.

In the embodiment, the cannula 3 is preferably fixed to a front end of the handle shell, the stylet 1 and the needle tube 2 penetrate through the cannula 3, and thus, the cannula 3 can ensure coaxality during movement of the stylet 1 and the needle tube 2.

In the embodiment, as shown in FIG.1 and FIG.2, the first spring-loading button 14 and the second spring-loading button 19 are arranged at the front end of the handle shell side by side from left to right. An extension arm, stretching into the handle shell, of the first spring-loading button 14 is connected to the first sliding block 6 so as to backwards push the first sliding block 6, an extension arm, stretching into the handle shell, of the second spring-loading button 19 is connected to the second sliding block 10 so as to backwards push the second sliding block 10, and the extension arm of the first spring-loading button 14 and the extension arm of the second spring-loading button 19 are arranged on a left side and a right side of the installation frame respectively. Accordingly, spring-loading operation is respectively and independently performed on the stylet and the needle tube by pressing the two spring-loading buttons, thus, operation is convenient, the structure is reasonable, and the spring-loading cocking action is reliable and stable.

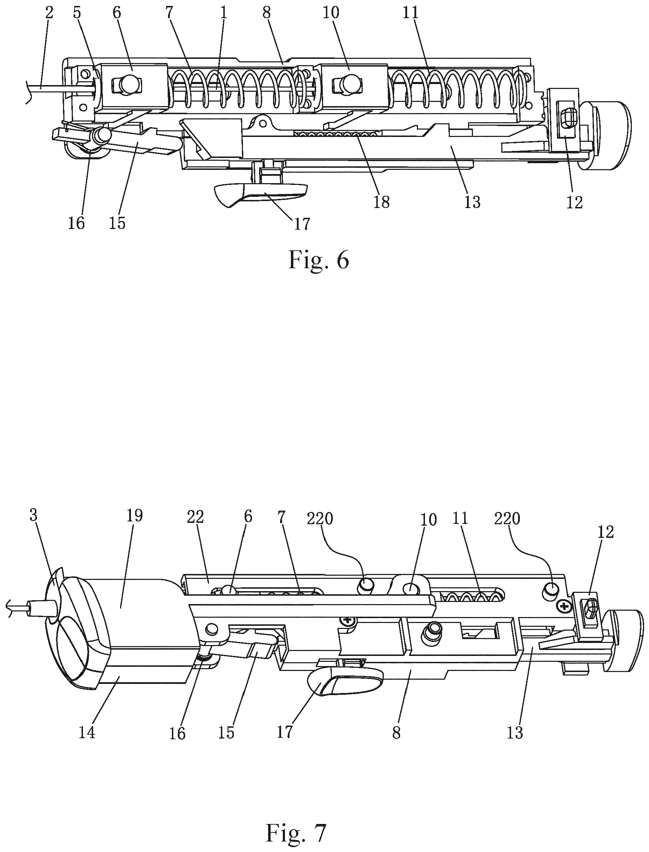

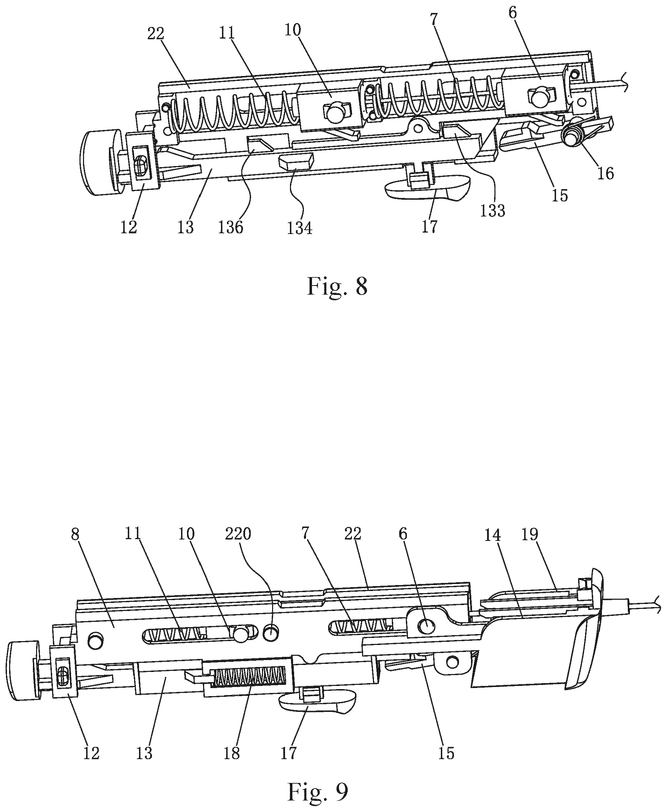

As shown in FIG.7 and FIG.9, preferably, the first sliding block 6 is provided with a first connecting portion 62, the first connecting portion 62 is connected to the extension arm of the first spring-loading button 14 after stretching out of a first slot hole 82 on the installation frame, and when the first sliding block 6 moves front and back, the first connecting portion 62 moves front and back along the first slot hole 82; and the second sliding block 10 is provided with a second connecting portion 102, the second connecting portion 102 is connected to the extension arm of the second spring-loading button 19 after stretching out of a second slot hole 84 on the installation frame, and when the second sliding block 10 moves front and back, the second connecting portion 102 moves front and back along the second slot hole 84. Accordingly, the structure is reasonable, installation is convenient, and connection is reliable. In other implementation modes, the connecting portions may be fixedly arranged or integrally formed on extension arms of spring-loading buttons and are connected with sliding blocks in an installation frame in an inserted manner after penetrating through slot holes on the installation frame. Preferably, the extension arm of the first spring-loading button 14 is matched with the first connecting portion 62 through a clamp groove or a connecting hole so as to backwards push the first sliding block 6, and the extension arm of the second spring-loading button 19 is matched with the second connecting portion 102 through a clamp groove or a connecting hole so as to backwards push the second sliding block 10. Furthermore, the clamp grooves limit the connecting portions from a front side and a rear side so that the spring-loading buttons and the corresponding sliding blocks synchronously move, and accordingly, a surgeon can visually know whether the biopsy device is in a spring-loading state or not, which avoids repeated operation. In other implementation modes, extension arms of the spring-loading buttons are connected to corresponding sliding blocks to implement spring-loading only during pressing for spring-loading, and after spring-loading is finished, the extension arms of the spring-loading buttons are separated from the corresponding sliding blocks, the sliding blocks and stylets or needles tube are independently cocked while the spring-loading buttons are not driven to move together, and to avoid random movement of the spring-loading buttons, springs may be arranged in a handle shell to reset the spring-loading buttons. Connecting portions may be in a cylinder or square column shape or any other shape capable of transmitting acting force.

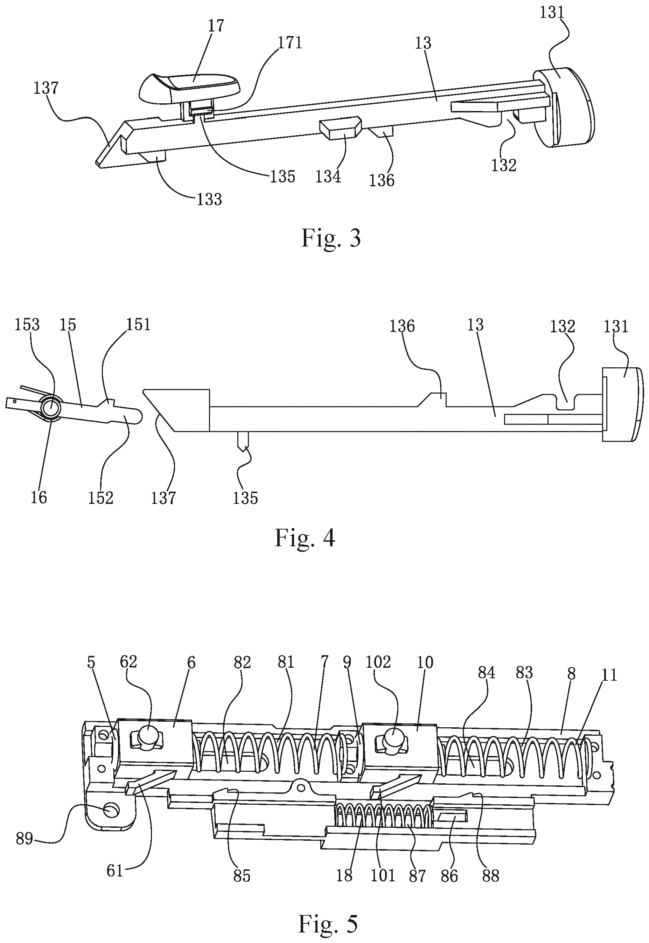

In the embodiment, as shown in FIG.5-FIG.9, a front portion of the installation frame is provided with a first slideway 81, the first sliding block 6 and the first cocking spring 7 are arranged in the first slideway 81, a rear portion of the installation frame is provided with a second slideway 83, the second sliding block 10 and the second cocking spring 11 are arranged in the second slideway 83, barrier portions are arranged at the front end of the first slideway 81, the rear end of the second slideway 83 and between the first slideway 81 and the second slideway 83, accordingly, the first sliding block 6 is limited to slide in the first slideway 81, and the second sliding block 10 is limited to slide in the second slideway 83. The installation frame is provided with a first locking portion 85 and a second locking portion 88, the first locking portion 85 is a convex lug, a front side of the first locking portion 85 is provided with a slope surface for promoting the first elastic barb 61 on the first sliding block 6 to be elastically contracted and deformed, a rear side of the first locking portion 85 is a clamping surface capable of being clamped and locked to a free end of the first elastic barb 61, the second locking portion 88 is a convex lug, a front side of the second locking portion 88 is provided with a slope surface for promoting the second elastic barb 101 on the second sliding block 10 to be elastically contracted and deformed, and a rear side of the second locking portion 88 is a clamping surface capable of being clamped and locked to a free end of the second elastic barb 101.

In the embodiment, as shown in FIG.3 and FIG.4, the cocking connecting rod 13 is provided with a first cocking portion 133 and a second cocking portion 136, the first cocking portion 133 and the second cocking portion 136 are both in a slope shape, and correspondingly, the first elastic barb 61 and the second elastic barb 101 are provided with slopes matched with the first cocking portion 133 and the second cocking portion 136 respectively; and when the cocking connecting rod 13 moves front and back for cocking, the second cocking portion 136 firstly thrusts the second elastic barb 101 on the second sliding block 10 to be elastically contracted and deformed so as to be separated from the second locking portion 88, accordingly, the second sliding block 10 connected to the stylet 1 is unlocked and cocked, then, the first cocking portion 133 thrusts the first elastic barb 61 of the first sliding block 6 to be elastically contracted and deformed so as to be separated from the first locking portion 85, and accordingly, the first sliding block 6 connected to the needle tube 2 is unlocked and cocked. Accordingly, the cocking portions are matched with the elastic barbs through slopes, which is more labor-saving and smoother in sliding. Linked sequential cocking of the two sliding blocks is achieved by setting specific locations of the two cocking portions on the cocking connecting rod and slope shapes, meanwhile, sequential cocking interval time of the stylet and the needle tube may be conveniently adjusted and designed, a desirable sampling effect is sufficiently guaranteed, the structure is simple, usage is convenient, and running is reliable.

In the embodiment, as shown in FIG.5 and FIG.8, the cocking connecting rod 13 is provided with a reset portion 134, a reset spring 18 is arranged between the reset portion 134 and the installation frame, and accordingly, after the cocking connecting rod 13 moves to be cocked, the cocking connecting rod 13 is driven by the reset spring 18 to reset. Preferably, the installation frame is provided with a reset spring space 87 and a fifth slideway 86, the reset spring 18 is limited within the reset spring space 87, a rear end of the reset spring space 87 communicates with the fifth slideway 86, the reset portion 134 on the cocking connecting rod 13 is inserted in the fifth slideway 86 and may move front and back, and when the cocking connecting rod 13 moves forwards, a front end of the reset spring 18 abuts against the installation frame, and a rear end of the reset spring 18 abuts against the reset portion 134 on the cocking connecting rod 13. Accordingly, the cocking connecting rod may rapidly reset, installation is convenient, front-back guided movement is stable and reliable, and movement cocking resistance of the cocking connecting rod 13 is lower, which facilitates surgeon operation.

In the embodiment, as shown in FIG.5 and FIG.9, the first slideway 81 and the second slideway 83 are jointly formed by the first installation frame 8 and the second installation frame 22, the cocking connecting rod 13 slides front and back along a sliding groove formed between the first installation frame 8 and the second installation frame 22, the first cocking portion 133 and the second cocking portion 136 are arranged on one side, close to the first slideway 81 and the second slideway 83, of the cocking connecting rod 13, the first locking portions/the first locking portion 85 are/is arranged on the first installation frame 8 and/or the second installation frame 22, located between the cocking connecting rod 13 and the first slideway 81; and the first locking portion 85 is located on two or one side of the first cocking portion 133, the first elastic barb 61 is arranged on one side, close to the cocking connecting rod 13, of the first sliding block 6, a front-back movement path of the first elastic barb 61 passes by the first locking portion 85 and partially coincides with a front-back movement path of the first cocking portion 133, and a width of a free end of the first elastic barb 61 is larger than that of the first cocking portion 133; the second locking portions/the second locking portion 88 are/is located on the first installation frame 8 and/or the second installation frame 22, located between the cocking connecting rod 13 and the second slideway 83; and the second locking portion 88 is located on two or one side of the second cocking portion 136, the second elastic barb 101 is arranged on one side, close to the cocking connecting rod 13, of the second sliding block 10, a front-back movement path of the second elastic barb 101 passes by the second locking portion 88 and partially coincides with a front-back movement path of the second cocking portion 136, and a width of a free end of the second elastic barb 101 is larger than that of the first cocking portion 136. Accordingly, not only can it facilitate clamping, locking and limiting of the elastic barbs and the locking portions, but also it can facilitate the cocking portions on the cocking connecting rod to thrust the elastic bars to be elastically contracted and deformed to implement unlocking and cocking, the structure is reasonable, assembling is convenient, and action running is reliable and stable.

In the embodiment, when the first sliding block 6 is in a spring-loaded locking state, the first locking portion 85 and the first cocking portion 133 are located on a front side and a rear side of the free end of the first elastic barb 61 respectively, and when the second sliding block 10 is in the spring-loaded locking state, the second locking portion 88 and the second cocking portion 136 are located on a front side and a rear side of the free end of the second elastic barb 101 respectively. The structure is simple and reasonable, actions are stable and reliable, manufacturing and assembling are convenient, and cost is low.

In the embodiment, a front end of the first sliding block 6 is provided with a first damping gasket 5, and the first damping gasket 5 has a function of buffering collisions, by the first sliding block 6, on the front barrier portions of the first slideway 81 so as to reduce noise and prolong service life; and a front end of the second sliding block 10 is provided with a second damping gasket 9, and the second damping gasket 9 has a function of buffering collisions, by the second sliding block 10, on the front barrier portions of the second slideway 83 so as to reduce noise and prolong service life.

In the embodiment, as shown in FIG.3-FIG.4 and FIG.6-FIG.9, the cocking connecting rod 13 is pushed by a rear cocking button 131 or a side cocking button 17 exposed out of the handle shell, and the rear cocking button 131 or the side cocking button 17 and the cocking connecting rod 13 are integrally formed or fixedly matched. Where, preferably, the rear cocking button 131 is integrally formed at a rear end of the cocking connecting rod 13, the side cocking button 17 is fixedly connected to the cocking connecting rod 13 through socket fit, and the side cocking button 17 is in limiting fit with the handle shell through a limiting clamp lug 171 and achieves front-back guided movement. In the embodiment, one side of the cocking connecting rod 13 is provided with a connecting portion 135, an extension arm, inserted into the handle shell, of the side cocking button 17 is provided with a clamp groove being in plug fit with the connecting portion 135, a left side and a right side of the side cocking button 17 are both provided with limiting clamp lugs 171, and the two limiting clamp lugs 171 are inserted into the handle shell and are in buckling and limiting fit with the handle shell.

A safety button 12 is further arranged to avoid false cocking caused by cocking button collisions after a product is spring-loaded, the safety button 12 is inserted in the handle shell and passes by one side of the cocking connecting rod 13, at least one end of the safety button 12 protrudes out of the handle shell, the safety button 12 is matched with a locking groove 132 in the cocking connecting rod 13 for limiting so as to lock and fix the cocking connecting rod 13, and the safety button 12 is moved to disengage it from the locking groove 132 so as to unlock the cocking connecting rod 13. In the embodiment, as shown in FIG.6 and FIG.7, the safety button 12 moves up and down in the handle shell, a guide piece is formed on a part, in the handle shell, of the safety button 12 so as to facilitate up-down movement guiding and limiting, the safety button 12 is provided with a locking portion, the cocking connecting rod 13 is provided with the corresponding locking groove 132, the locking portion is separated from the locking groove 132 by upwards moving the safety button 12, and the cocking connecting rod 13 can move front and back for cocking; and the locking portion is clamped in the locking groove 132 for limiting and locking by downwards moving the safety button 12, and then the cocking connecting rod 13 cannot move front and back.

To adjust a sampling length and achieve multi-sampling-length biopsy sampling on one biopsy device, a sampling length adjusting mechanism is further arranged, and as shown in FIG.4 and FIG.6, includes a sampling length adjusting component 15, the sampling length adjusting component 15 is provided with a hook boss 151, the sampling length adjusting component can move and at least has a locking state and an unlocking state, when the sampling length adjusting component is in the locking state, the hook boss 151 is arranged on the front-back movement path of the first elastic barb 61 on the first sliding block 6, and the first elastic barb 61 can be hooked with the hook boss 151 for positioning so as to achieve spring-loaded locking, and when the first sliding block 6 is locked at the position, only part of a sampling channel of the stylet 1 can be exposed out of the needle tube 2 so as to implement incomplete-length sampling; and when the sampling length adjusting component is in the unlocking state, the hook boss 151 leaves the front-back movement path of the first elastic barb 61 on the first sliding block 6, and the first elastic barb 61 can freely pass without obstruction.

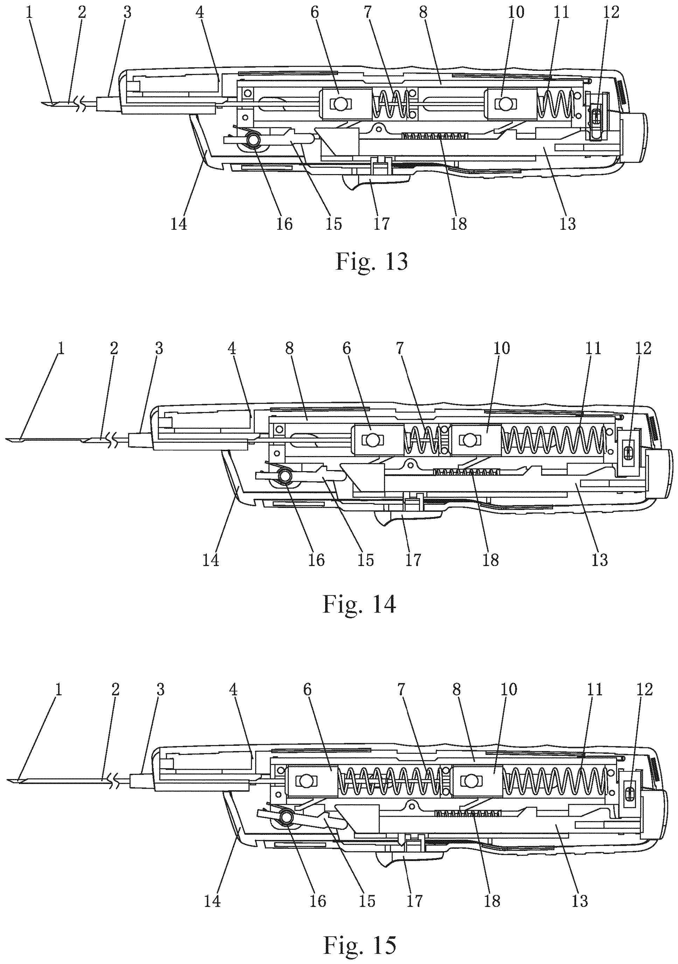

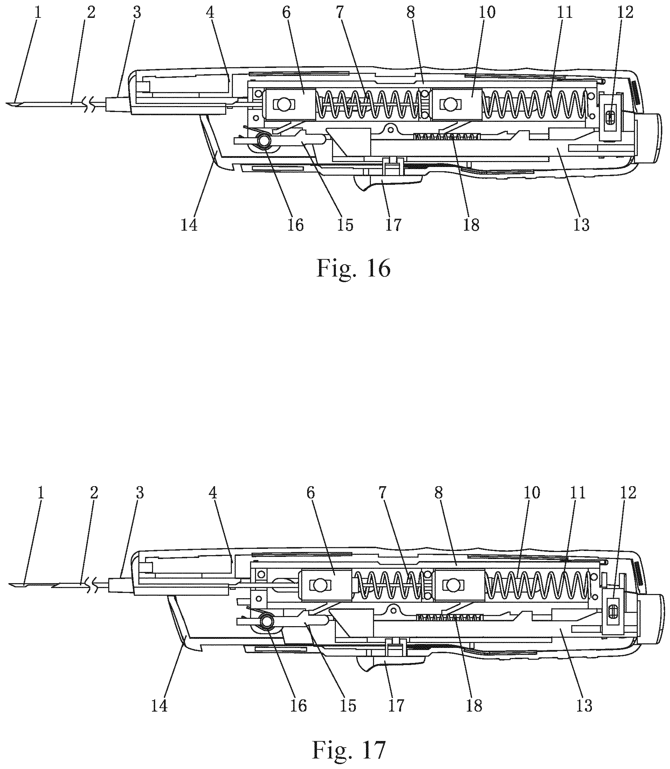

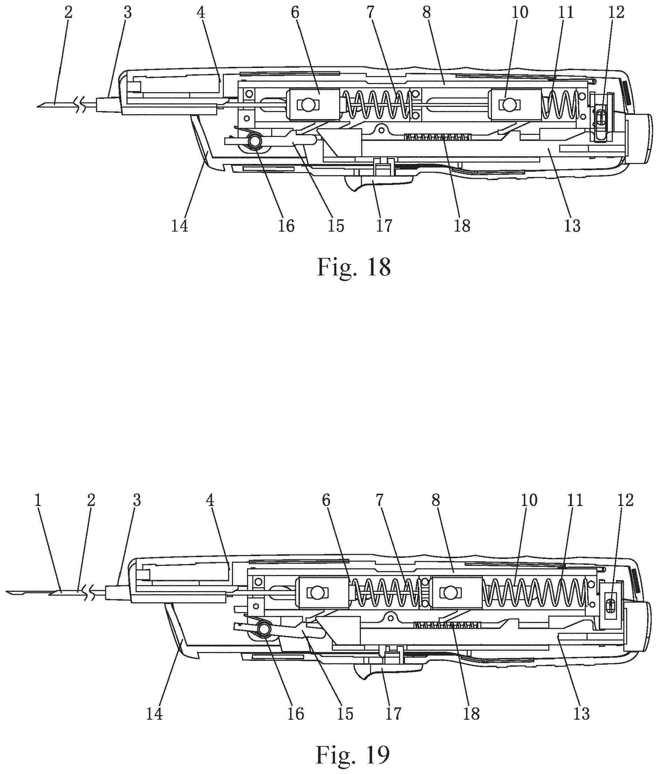

In the embodiment, as shown in FIG.4, FIG.6 and FIG.7, the sampling length adjusting component 15 is hinged to the installation frame, and can swing up and down, the sampling length adjusting component 15 is provided with the hook boss 151 and a release portion 152, an elastic member 16 makes the sampling length adjusting component 15 rotate and makes the hook boss 151 get onto the front-back movement path of the first elastic barb 61 on the first sliding block 6 (namely, the elastic member makes the sampling length adjusting component restore to the locking state from the unlocking state); as shown in FIG.17-FIG.18, the first elastic barb 61 of the first sliding block 6 moves backwards to be hooked with the hook boss 151 for positioning so as to achieve spring-loaded locking, and when the first sliding block 6 is locked at the position, only part of the sampling channel of the stylet 1 can be exposed out of the needle tube 2 so as to implement incomplete-length sampling (or half-length sampling); and as shown in FIG.15 and FIG.19, the front end of the cocking connecting rod 13 is provided with a thrust portion 137, when the cocking connecting rod 13 moves forwards, the thrust portion 137 abuts against the release portion 152 and can make the sampling length adjusting component 15 rotate so as to enable the hook boss 151 to retreat from the front-back movement path of the first elastic barb 61, accordingly, during incomplete-length sampling, the cocking connecting rod 13 can release clamping and locking, by the hook boss 151 on the first elastic barb 61 so as to achieve releasing and cocking of the first sliding block 6, and during full-length sampling, the cocking connecting rod 13 can prevent the first elastic barb 61 from making contact with the hook boss 151 in a cocking forward movement process. Accordingly, a spring-loading and locking position of the first sliding block 6 can be selected by adjusting a pressing length of the first spring-loading button 14, thereby adjusting the biopsy device sampling length (sampling volume) without manual operation of sampling length buttons for setting in advance, the structure is simple and reasonable, and operation is convenient and reliable.

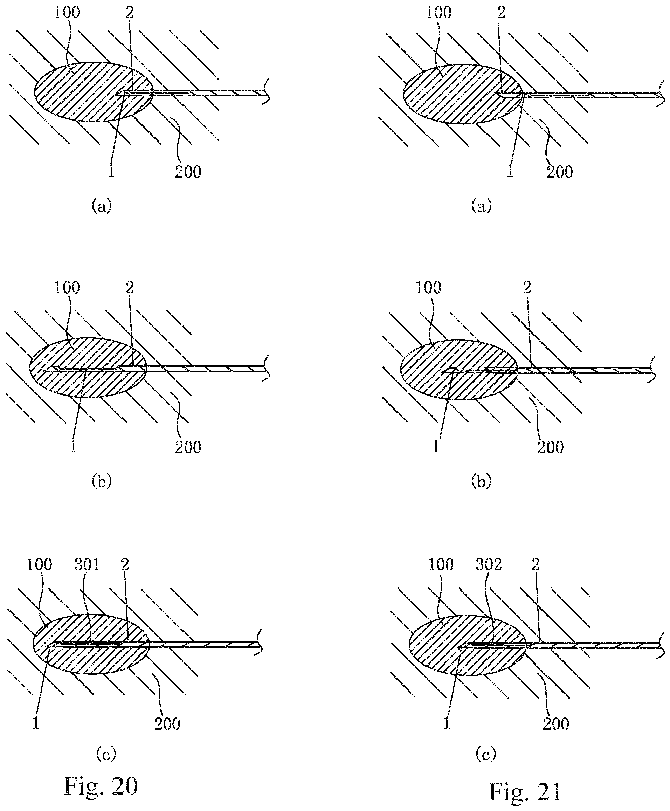

The "full-length sampling" also called as whole-channel sampling means that a whole sampling channel of the stylet 1 punctures into a target sampling tissue to obtain a biopsy specimen, and a specific process is as below:
after the stylet 1 and the needle tube 2 are spring-loaded, the whole sampling channel of the stylet 1 is contained in the needle tube 2, and a front end of the stylet and a front end of the needle tube puncture into the target sampling tissue 100, shown in FIG.20(a); the stylet 1 is firstly cocked to puncture into the target sampling tissue 100, the whole sampling channel in the stylet 1 is exposed out of the needle tube 2, and the whole sampling channel is filled with the target sampling tissue 100, shown in FIG.20(b); and then, the needle tube 2 is cocked, the biopsy specimen 301 is incised, by the needle tube 2, from the target puncture tissue 100 and stored in the sampling channel of the stylet, shown in FIG.20(c).

The "incomplete-length sampling" also called as partial-length sampling or half-length sampling means that a part of sampling channel of the stylet 1 punctures into the target sampling tissue to obtain the biopsy specimen, and a specific process is as below:
after the stylet 1 and the needle tube 2 are spring-loaded, the whole sampling channel of the stylet 1 is contained in the needle tube 2, and the front end of the stylet and the front end of the needle tube puncture into the target sampling tissue 100, shown in FIG.21(a); the stylet 1 is firstly cocked to puncture into the target sampling tissue 100, a part of sampling channel (commonly, half length of the whole sampling channel) in the stylet 1 is exposed out of the needle tube 2, and the part of sampling channel is filled with the target sampling tissue 100, shown in FIG. 21(b); and then, the needle tube 2 is cocked, a biopsy specimen 302 is incised, by the needle tube 2, from the target puncture tissue 100 and stored in the sampling channel of the stylet, shown in FIG. 21(c), where the biopsy specimen 302 is obviously shorter than the biopsy specimen sampled in full length shown in FIG.20(c),and requirements for different sampling volumes can be met.

In the embodiment, as shown in FIG.4-FIG.6, the sampling length adjusting component 15 is in a rod shape extending front and back, a hinge portion 153 is arranged on a left side and a right side of the sampling length adjusting component 15, and the hinge portion 153 inserted in a hinge hole 89 on the installation frame so that the sampling length adjusting component 15 can be hinged to the installation frame; and the elastic member 16 is a torsion spring sleeving the hinge portion 153, one end of the torsion spring abuts against the installation frame, the other end of the torsion spring abuts against a front end of the sampling length adjusting component 15, and the release portion 152 is located at a rear end of the sampling length adjusting component 15. The structure is simple, restoring is reliable, and installation is convenient. In other implementation modes, an elastic member 16 may be a compression spring, an elastic clip, or a rubber member, or the like.

In the embodiment, as shown in FIG.4-FIG.7, the thrust portion 137 is located on one side of the first cocking portion 133 so as to avoid contact between the thrust portion 137 and the first elastic barb 61 on the first sliding block 6; and a contact surface, making contact with the release portion 152, of the thrust portion 137 is a slope or a cambered surface, and a contact surface, making contact with the thrust portion 137, of the release portion 152 is a slope or a cambered surface. The structure is simple and reasonable, motions are stable and reliable, unlocking is convenient, and manufacturing cost is low. Further preferably, the thrust portion 137 and the first locking portion 85 are located on a left side and a right side of the first cocking portion 133 respectively, the thrust portion 137 is located on a left side or a right side of the front-back movement path of the first elastic barb 61, and the width of the free end of the first elastic barb 61 is larger than the width of the thrust portion 137. The structure is more reasonable, and assembling is more convenient.

In other implementation modes, a plurality of sampling length adjusting components 15 may be arranged to obtain biopsy specimens different in length.

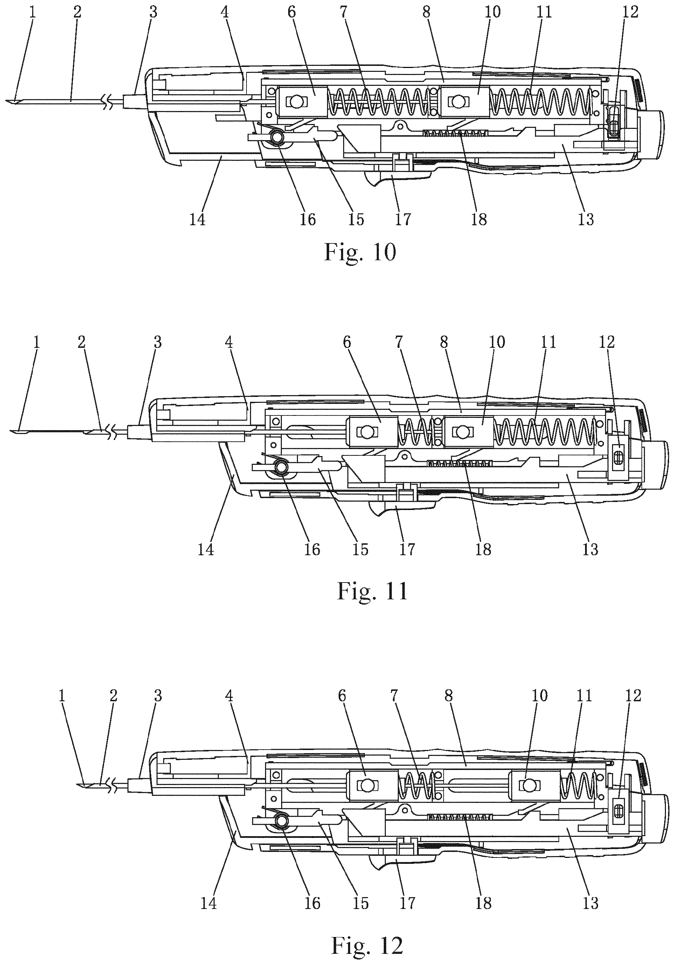

When the biopsy device is not used, as shown in FIG. 10, the first sliding block 6 and the second sliding block 10 are not in a spring-loading state, the sampling channel of the stylet 1 is contained in the needle tube 2, the safety button 12 locks the cocking connecting rod 13, and the rear cocking button 131 and the side cocking button 17 cannot be pressed.

A spring-loading cocking process/method for full-length sampling by the biopsy device includes the following steps:
3) spring-loading,
   As shown in FIG.11-FIG.12, the first spring-loading button 14 and the second spring-loading button 19 are respectively pressed; the first sliding block 6 is driven by the first spring-loading button 14 to move backwards and compress the first cocking spring 7 until the first elastic barb 61 on the first sliding block 6 is locked, and the first sliding block 6 and the needle tube 2 finish spring-loading; the second sliding block 10 is driven by the second spring-loading button 19 to move backwards and compress the second cocking spring 11 until the second elastic barb 101 on the second sliding block 10 is locked, and the second sliding block 10 and the stylet 1 finish spring-loading; and at the time, the safety button 12 is preferably selected to lock the cocking connecting rod 13 so as to avoid false cocking.
4) opening a safety,
   As shown in FIG. 13, the safety button 12 is opened to unlock the cocking connecting rod 13.
5) cocking,
   The rear cocking button 131 is pressed or the side cocking button 17 is forwards pushed to drive the cocking connecting rod 13 to move forwards; and
   firstly, as shown in FIG.14,the cocking connecting rod 13 promotes, by the second cocking portion 136, the second elastic barb 101 to be elastically deformed and separated from the second locking portion 88, thereby unlocking the second sliding block 10, and the second sliding block 10 is pushed by the second cocking spring 11 to move forwards to finish unlocking and cocking of the second sliding block 10 and the stylet 1; and at the same time, the first cocking portion 133 of the cocking connecting rod 13 does not make contact with the first elastic barb 61 on the first sliding block 6 to maintain the locking state of the first sliding block 6; and accordingly, the sampling channel of the stylet 1 is exposed out of the front end of the needle tube 2.

Then, along with continuous forward movement of the cocking connecting rod 13, as shown in FIG.15, the cocking connecting rod 13 promotes, by the first cocking portion 133, the first elastic barb 61 to be elastically deformed and separated from the first locking portion 85 so as to unlock the first sliding block 6, the first sliding block 6 is pushed by the first cocking spring 7 to move forwards so as to finish unlocking and cocking of the first sliding block 6 and the needle tube 2, and the needle tube 2 moves forwards so as to accommodate the sampling channel of the stylet 1 in the front end; and at the same time, the thrust portion 137 of the cocking connecting rod 13 abuts against the release portion 152 and promotes the sampling length adjusting component 15 to rotate so as to make the hook boss 151 retreat from the front-back movement path of the first elastic barb 61, thereby avoiding contact between the first elastic barb 61 and the hook boss 151 in a cocking forward movement process.

Finally, the rear cocking button 131 and the side cocking button 17 are released, as shown in FIG. 16, the cocking connecting rod 13 is pushed by the reset spring 18 to move backwards and reset, and the sampling length adjusting component 15 resets under the action of the elastic member 16.

A spring-loading cocking process/method for incomplete-length sampling by the biopsy device includes the following steps:
1) incomplete-length spring-loading,
   The first spring-loading button 14 and the second spring-loading button 19 are respectively pressed; the first sliding block 6 is driven by the first spring-loading button 14 to move backwards and compress the first cocking spring 7 until the first elastic barb 61 on the first sliding block 6 is locked with the hook boss 151 on the sampling length adjusting component 15, and the first sliding block 6 and the needle tube 2 finish spring-loading, shown in FIG. 17; the second sliding block 10 is driven by the second spring-loading button 19 to move backwards and compress the second cocking spring 11 until the second elastic barb 101 on the second sliding block 10 is locked, and the second sliding block 10 and the stylet 1 finish spring-loading, shown in FIG.18; and at the time, the safety button 12 is preferably selected to lock the cocking connecting rod 13 so as to avoid false cocking.
2) opening a safety,
   The safety button 12 is opened to unlock the cocking connecting rod 13.
3) cocking,
   The rear cocking button 131 is pressed or the side cocking button 17 is forwards pushed to drive the cocking connecting rod 13 to move forwards; and
   firstly, the cocking connecting rod 13 promotes, by the second cocking portion 136, the second elastic barb 101 to be elastically deformed and separated from the second locking portion 88, thereby unlocking the second sliding block 10, and the second sliding block 10 is pushed by the second cocking spring 11 to move forwards to finish unlocking and cocking of the second sliding block 10 and the stylet 1; and at the same time, the first cocking portion 133 and the thrust portion 137 of the cocking connecting rod 13 do not make contact with the first elastic barb 61 on the first sliding block 6 to maintain the locking state of the first sliding block 6; and accordingly, a part of the sampling channel of the stylet 1 is exposed out of the front end of the needle tube 2.

Then, as shown in FIG. 19, along with continuous forward movement of the cocking connecting rod 13, the thrust portion 137 at the front end of the cocking connecting rod 13 abuts against the release portion 152 and can promote the sampling length adjusting component 15 to rotate so as to make the hook boss 151 retreat from the front-back movement path of the first elastic barb 61, thereby releasing clamping and locking, by the hook boss 151, on the first elastic barb 61, the first sliding block 6 is pushed by the first cocking spring 7 to move forwards to finish unlocking and cocking of the first sliding block 6 and the needle tube 2, and the needle tube 2 moves forwards to accommodate the sampling channel of the stylet 1 in the front end.

Finally, the rear cocking button 131 and the side cocking button 17 are released, the cocking connecting rod 13 is pushed by the reset spring 18 to move backwards and reset, and the sampling length adjusting component 15 resets under the action of the elastic member 16, shown in FIG. 16..

In the description of the specification, descriptions of reference terms "one embodiment", "some embodiments", "example", "specific example" or "some examples" or the like imply that specific features, structures, materials or characteristics combined with the embodiment or the example are included in at least one embodiment or example of the present disclosure. In the specification, schematic statements of the above terms are not necessarily specific to the same embodiment or example. Moreover, the described specific features, structures, materials or characteristics can be properly combined in any one or more embodiments or examples.

Although the embodiment of the present disclosure is shown and described above, it is to be understood that the above embodiment is illustrative and cannot be understood as limitation on the present disclosure, and ordinary skill in the art can change, modify, replace and transform the above embodiment in the scope of the present disclosure without departing from the principle and purpose of the present disclosure. Any modification, equivalent replacement, improvement, etc., made within the spirit and principle of the present disclosure shall fall within the scope of protection of the present disclosure.

## Claims

1. A biopsy device, comprising a stylet (1), a needle tube (2) and an operating handle for controlling the stylet and the needle tube to perform biopsy sampling, **characterized in that** the operating handle comprises an installation frame internally provided with a first sliding block (6), a first cocking spring (7), a second sliding block (10) and a second cocking spring (11), a rear end of the needle tube (2) is fixedly connected to the first sliding block (6), a rear end of the stylet (1) penetrates out of the needle tube (2) and the first sliding block(6) and then is fixedly connected to the second sliding block (10), a front end of the first cocking spring (7) abuts against the first sliding block (6), a rear end of the first cocking spring (7) abuts against the installation frame, a front end of the second cocking spring (11) abuts against the second sliding block (10), and a rear end of the second cocking spring (11) abuts against the installation frame; and the installation frame is further provided with a spring-loading cocking mechanism for driving the first sliding block (6) and the second sliding block (10) to be moved backwards, locked and positioned and compressing the first cocking spring (7) and the second cocking spring (11) for potential storage, and a cocking-unlocking mechanism for releasing locking and positioning of the first sliding block (6) and/or the second sliding block (10).

2. The biopsy device according to claim 1, **characterized in that** the spring-loading cocking mechanism comprises a first spring-loading button (14) and a second spring-loading button (19), the first spring-loading button (14) is connected to the first sliding block (6) and may drive the first sliding block (6) to move backwards, the first sliding block (6)is provided with a first elastic barb (61), after moving backwards in place, the first sliding block (6) is hooked and locked, by the first elastic barb (61), with a first locking portion (85) arranged on the installation frame so as to achieve spring-loading, the second spring-loading button (19) is connected to the second sliding block (10) and may drive the second sliding block (10) to move backwards, the second sliding block (10) is provided with a second elastic barb (101), and after moving backwards in place, the second sliding block (10) is hooked and locked, by the second elastic barb (101), with a second locking portion (88) arranged on the installation frame so as to achieve spring-loading.

3. The biopsy device according to claim 2, **characterized in that** a front portion of the installation frame is provided with a first slideway (81), the first sliding block (6) and the first cocking spring (7) are arranged in the first slideway (81), a rear portion of the installation frame is provided with a second slideway (83), the second sliding block (10) and the second cocking spring (11) are arranged in the second slideway (83), barrier portions are arranged at a front end of the first slideway (81), a rear end of the second slideway (83) and between the first slideway (81) and the second slideway (83), accordingly, the first sliding block (6) is limited to slide in the first slideway (81), and the second sliding block (10) is limited to slide in the second slideway (83); the installation frame is provided with the first locking portion (85) and the second locking portion (88), the first locking portion (85) is a convex lug, a front side of the first locking portion (85) is provided with a slope surface for promoting the first elastic barb (61) on the first sliding block (6) to be elastically contracted and deformed, a rear side of the first locking portion (85) is a clamping surface capable of being clamped and locked to a free end of the first elastic barb (61), the second locking portion (88) is a convex lug, a front side of the second locking portion (88) is provided with a slope surface for promoting the second elastic barb (101) on the second sliding block (10) to be elastically contracted and deformed, and a rear side of the second locking portion (88) is a clamping surface capable of being clamped and locked to a free end of the second elastic barb (101); a handle shell is fixed to the exterior of the installation frame, the first spring-loading button (14) and the second spring-loading button (19) are arranged at a front end of the handle shell side by side from left to right, an extension arm, stretching into the handle shell, of the first spring-loading button (14) is connected to the first sliding block (6) so as to backwards push the first sliding block (6), an extension arm, stretching into the handle shell, of the second spring-loading button (19) is connected to the second sliding block (10) so as to backwards push the second sliding block (10), and the extension arm of the first spring-loading button (14) and the extension arm of the second spring-loading button (19) are arranged on a left side and a right side of the installation frame respectively.

4. The biopsy device according to claim 1, **characterized in that** the cocking-unlocking mechanism comprises a cocking connecting rod (13), the cocking connecting rod (13) is arranged on the installation frame and can move front and back, the cocking connecting rod (13) is provided with a first cocking portion (133) and a second cocking portion (136), the cocking connecting rod (13) promotes, by the first cocking portion (133), the first elastic barb (61) to be elastically deformed and separated from the first locking portion (85) so as to unlock the first sliding block (6), and the cocking connecting rod (13) promotes, by the second cocking portion (136), the second elastic barb (101) to be elastically deformed and separated from the second locking portion (88) so as to unlock the second sliding block (10).

5. The biopsy device according to claim 4, **characterized in that** the first cocking portion (133) and the second cocking portion (136) are both in a slope shape, and correspondingly, the first elastic barb (61) and the second elastic barb (101) are provided with slopes matched with the first cocking portion (133) and the second cocking portion (136) respectively; when the cocking connecting rod (13) moves front and back for cocking, the second cocking portion (136) firstly thrusts the second elastic barb (101) on the second sliding block (10) to be elastically contracted and deformed and be separated from the second locking portion (88), accordingly, the second sliding block (10) connected to the stylet (1) is unlocked and cocked, then, the first cocking portion (133) thrusts the first elastic barb (61) of the first sliding block (6) to be elastically contracted and deformed and be separated from the first locking portion (85), and accordingly, the first sliding block (6) connected to the needle tube (2) is unlocked and cocked; the cocking connecting rod (13) is provided with a reset portion (134), and a reset spring (18) is arranged between the reset portion (134) and the installation frame; and the installation frame is provided with a reset spring space (87) and a fifth slideway (86), the reset spring (18) is limited within the reset spring space (87), a rear end of the reset spring space (87) communicates with the fifth slideway (86), the reset portion (134) on the cocking connecting rod (13) is inserted in the fifth slideway (86) and may move front and back, and when the cocking connecting rod (13) moves forwards, a front end of the reset spring (18) abuts against the installation frame, and a rear end of the reset spring (18) abuts against the reset portion (134) on the cocking connecting rod (13).

6. The biopsy device according to claim 1, **characterized in that** a first damping gasket (5) is arranged at a front end of the first sliding block (6), and a second damping gasket (9) is arranged at a front end of the second first sliding block (10).

7. The biopsy device according to claim 1, **characterized in that** a cocking connecting rod (13) is pushed by a rear cocking button (131) or a side cocking button (17) exposed out of a handle shell, and the rear cocking button (131) or the side cocking button (17) and the cocking connecting rod (13) are integrally formed or fixedly matched.

8. The biopsy device according to claim 1, **characterized in that** a safety button (12) is further arranged, the safety button (12) is inserted in a handle shell and passes by one side of a cocking connecting rod (13), wherein at least one end of the safety button (12) protrudes out of the handle shell, the safety button (12) is matched with a locking groove (132) in the cocking connecting rod (13) for limiting so as to lock and fix the cocking connecting rod (13), and the safety button (12) is moved not to be matched with the locking groove(132) so as to unlock the cocking connecting rod (13).

9. The biopsy device according to claim 1, further provided with a sampling length adjusting mechanism comprising a sampling length adjusting component (15), **characterized in that** the sampling length adjusting component (15) is provided with a hook boss (151), the sampling length adjusting component (15) can move and at least has a locking state and an unlocking state, the hook boss (151) is arranged on a front-back movement path of a first elastic barb (61) on the first sliding block (6) when the sampling length adjusting component is in the locking state, and the first elastic barb (61) can be hooked with the hook boss (151) for positioning so as to achieve spring-loaded locking, and when the first sliding block (6) is locked at the position, only part of a sampling channel of the stylet (1) can be exposed out of the needle tube (2) so as to implement incomplete-length sampling; when the sampling length adjusting component is in the unlocking state, the hook boss (151) leaves the front-back movement path of the first elastic barb (61) on the first sliding block (6), and the first elastic barb (61) can freely pass without obstruction; the sampling length adjusting component (15) is hinged to the installation frame, and can swing up and down, the sampling length adjusting component (15) is provided with the hook boss (151) and a release portion (152), and an elastic member (16) makes the sampling length adjusting component (15) rotate and makes the hook boss (151) get onto the front-back movement path of the first elastic barb (61) on the first sliding block (6); the first elastic barb (61) of the first sliding block (6) moves backwards to be hooked with the hook boss (151) for positioning so as to achieve spring-loaded locking, and when the first sliding block (6) is locked at the position, only part of the sampling channel of the stylet (1) can be exposed out of the needle tube (2); and the front end of the cocking connecting rod (13) is provided with a thrust portion (137), when the cocking connecting rod (13) moves forwards, the thrust portion (137) abuts against the release portion (152) and can promote the sampling length adjusting component (15) to rotate so as to enable the hook boss (151) to retreat from the front-back movement path of the first elastic barb (61).

10. A spring-loading cocking method for the biopsy device according to any one of claims 1-9, comprising the following steps:
1) spring-loading,
wherein the first spring-loading button (14) and the second spring-loading button (19) are respectively pressed; the first sliding block (6) is driven by the first spring-loading button (14) to move backwards and compress the first cocking spring (7) until the first elastic barb (61) on the first sliding block (6) is locked, and the first sliding block (6) and the needle tube (2) finish spring-loading; and the second sliding block (10) is driven by the second spring-loading button (19) to move backwards and compress the second cocking spring (11) until the second elastic barb (101) on the second sliding block (10) is locked, and the second sliding block (10) and the stylet (1) finish spring-loading; and
2) cocking,
wherein the rear cocking button (131) is pressed or the side cocking button (17) is forwards pushed to drive the cocking connecting rod (13) to move forwards;
firstly, the cocking connecting rod (13) promotes, by the second cocking portion (136), the second elastic barb (101) to be elastically deformed and separated from the second locking portion (88) so as to unlock the second sliding block (10), and the second sliding block (10) is pushed by the second cocking spring (11) to move forwards to finish unlocking and cocking of the second sliding block (10) and the stylet (1); at the same time, the first cocking portion (133) of the cocking connecting rod (13) does not make contact with the first elastic barb (61) on the first sliding block (6) to maintain the locking state of the first sliding block (6); accordingly, the sampling channel of the stylet (1) is exposed out of the front end of the needle tube (2);
then, along with continuous forward movement of the cocking connecting rod (13), the cocking connecting rod (13) promotes, by the first cocking portion (133), the first elastic barb (61) to be elastically deformed and separated from the first locking portion (85) so as to unlock the first sliding block (6), the first sliding block (6) is pushed by the first cocking spring (7) to move forwards so as to finish unlocking and cocking of the first sliding block (6) and the needle tube (2), and the needle tube (2) moves forwards so as to accommodate the sampling channel of the stylet (1) in the front end; at the same time, the thrust portion (137) of the cocking connecting rod (13) abuts against the release portion (152) and promotes the sampling length adjusting component (15) to rotate to make the hook boss (151) retreat from the front-back movement path of the first elastic barb (61) so as to avoid contact between the first elastic barb (61) and the hook boss (151) in a cocking forward movement process; and
finally, the rear cocking button (131) and the side cocking button (17) are released, the cocking connecting rod (13) is pushed by the reset spring (18) to move backwards and reset, and the sampling length adjusting component (15) resets under the action of the elastic member (16).

11. A cocking-unlocking mechanism of a biopsy device, comprising an installation frame, **characterized in that** a first sliding block (6), a first cocking spring (7), a second sliding block (10), a second cocking spring (11) and a cocking connecting rod (13) are arranged in the installation frame, the first sliding block (6) is fixedly connected to a rear end of a needle tube (2), a front end of the first cocking spring (7) abuts against the first sliding block (6), a rear end of the first cocking spring (7) abuts against the installation frame, the first sliding block (6) is provided with a first elastic barb (61), and after moving backwards in place, the first sliding block (6) is hooked and locked, by the first elastic barb (61), with a first locking portion (85) arranged on the installation frame so as to achieve spring-loading; the second sliding block (10) is fixedly connected to a rear end of a stylet (1), a front end of the second cocking spring (11) abuts against the second sliding block (10), a rear end of the second cocking spring (11) abuts against the installation frame, the second sliding block (10) is provided with a second elastic barb (101), and after moving backwards in place, the second sliding block (10) is hooked and locked, by the second elastic barb (101), with a second locking portion (88) arranged on the installation frame so as to achieve spring-loading; and wherein, the first sliding block (6) and the second sliding block (10) are respectively in front-back sliding connection to the installation frame and are arranged front and back; the cocking connecting rod (13) is arranged on the installation frame, can move front and back, the cocking connecting rod (13) is provided with a first cocking portion (133) and a second cocking portion (136), the cocking connecting rod (13) promotes, by the first cocking portion (133), the first elastic barb (61) to be elastically deformed and separated from the first locking portion (85) so as to unlock the first sliding block (6), and the cocking connecting rod (13) promotes, by the second cocking portion (136), the second elastic barb (101) to be elastically deformed and separated from the second locking portion (88) so as to unlock the second sliding block (10).

12. The cocking-unlocking mechanism of the biopsy device according to claim 11, **characterized in that** the first locking portion (85) is a convex lug, a front side of the first locking portion (85) is provided with a slope surface for promoting the first elastic barb (61) on the first sliding block (6) to be elastically contracted and deformed, a rear side of the first locking portion (85) is a clamping surface capable of being clamped and locked to a free end of the first elastic barb (61), the second locking portion (88) is a convex lug, a front side of the second locking portion (88) is provided with a slope surface for promoting the second elastic barb (101) on the second sliding block (10) to be elastically contracted and deformed, and a rear side of the second locking portion (88) is a clamping surface capable of being clamped and locked to a free end of the second elastic barb (101).

13. The cocking-unlocking mechanism of the biopsy device according to claim 12, **characterized in that** the cocking connecting rod (13) is provided with the first cocking portion (133) and the second cocking portion (136), the first cocking portion (133) and the second cocking portion (136) are both in a slope shape, and correspondingly, the first elastic barb (61) and the second elastic barb (101) are provided with slopes matched with the first cocking portion (133) and the second cocking portion (136) respectively; when the cocking connecting rod (13) moves front and back for cocking, the second cocking portion (136) firstly thrusts the second elastic barb (101) on the second sliding block (10) to be elastically contracted and deformed and be separated from the second locking portion (88), accordingly, the second sliding block (10) connected to the stylet (1) is unlocked and cocked, then, the first cocking portion (133) thrusts the first elastic barb (61) of the first sliding block (6) to be elastically contracted and deformed and be separated from the first locking portion (85), and accordingly, the first sliding block (6) connected to a needle tube (2) is unlocked and cocked.

14. The cocking-unlocking mechanism of the biopsy device according to claim 11, **characterized in that** the cocking connecting rod (13) is provided with a reset portion (134), and a reset spring (18) is arranged between the reset portion (134) and the installation frame; wherein, the installation frame is provided with a reset spring space (87) and a fifth slideway (86), the reset spring (18) is limited within the reset spring space (87), a rear end of the reset spring space (87) communicates with the fifth slideway (86), the reset portion (134) on the cocking connecting rod (13) is inserted in the fifth slideway (86) and can move front and back, and when the cocking connecting rod (13) moves forwards, a front end of the reset spring (18) abuts against the installation frame, and a rear end of the reset spring (18) abuts against the reset portion (134) on the cocking connecting rod (13).

15. The cocking-unlocking mechanism of the biopsy device according to claim 11, **characterized in that** a front portion of the installation frame is provided with a first slideway (81), the first sliding block (6) and the first cocking spring (7) are arranged in the first slideway (81), a rear portion of the installation frame is provided with a second slideway (83), the second sliding block (10) and the second cocking spring (11) are arranged in the second slideway (83), barrier portions are arranged at a front end of the first slideway (81), a rear end of the second slideway (83) and between the first slideway (81) and the second slideway (83), accordingly, the first sliding block (6) is limited to slide in the first slideway (81), and the second sliding block (10) is limited to slide in the second slideway (83); and wherein, a first damping gasket (5) is arranged at a front end of the first sliding block (6), and a second damping gasket (9) is arranged at a front end of the second sliding block (10).

16. The cocking-unlocking mechanism of the biopsy device according to claim 15, **characterized in that** the first slideway (81) and the second slideway (83) are jointly formed by a first installation frame (8) and a second installation frame (22), the cocking connecting rod (13) slides front and back along a sliding groove formed between the first installation frame (8) and the second installation frame (22), the first cocking portion (133) and the second cocking portion (136) are arranged on one side, close to the first slideway (81) and the second slideway (83), of the cocking connecting rod (13), the first locking portions and/or the first locking portion (85) are/is arranged on the first installation frame (8) and/or the second installation frame (22), located between the cocking connecting rod (13) and the first slideway (81), and the first locking portion (85) is located two or one side of the first cocking portion (133), the first elastic barb (61)is arranged on one side, close to the cocking connecting rod (13), of the first sliding block (6), a front-back movement path of the first elastic barb (61) passes by the first locking portion (85) and partially coincides with a front-back movement path of the first cocking portion (133), and a width of a free end of the first elastic barb (61) is larger than that of the first cocking portion (133); second locking portions/the second locking portion (88) are/is located on the first installation frame (8) and/or the second installation frame (22), located between the cocking connecting rod (13) and the second slideway (83), and the second locking portion (88) is located on two or one side of the second cocking portion (136), the second elastic barb (101)is arranged on one side, close to the cocking connecting rod (13), of the second sliding block (10), a front-back movement path of the second elastic barb (101) passes by the second locking portion (88) and partially coincides with a front-back movement path of the second cocking portion (136), and a width of a free end of the second elastic barb (101) is larger than that of the first cocking portion (136).

17. The cocking-unlocking mechanism of the biopsy device according to claim 16, **characterized in that** when the first sliding block (6) is in a spring-loaded locking state, the first locking portion (85) and the first cocking portion (133) are located on a front side and a rear side of the free end of the first elastic barb (61) respectively, and when the second sliding block (10) is in a spring-loaded locking state, the second locking portion (88) and the second cocking portion (136) are located on a front side and a rear side of the free end of the second elastic barb (101) respectively.

18. The cocking-unlocking mechanism of the biopsy device according to claim 11, **characterized in that** the cocking connecting rod (13) is pushed by a rear cocking button (131) or a side cocking button (17) exposed out of a handle shell, and the rear cocking button (131) or the side cocking button (17) and the cocking connecting rod (13) are integrally formed or fixedly matched.

19. The cocking-unlocking mechanism of the biopsy device according to claim 11, **characterized in that** a safety button (12) is further arranged, the safety button (12) is inserted in a handle shell and passes by one side of the cocking connecting rod (13), wherein at least one end of the safety button (12) protrudes out of the handle shell, the safety button (12) is matched with a locking groove (132) in the cocking connecting rod (13) for limiting so as to lock and fix the cocking connecting rod (13), and the safety button (12) is moved not to be matched with the locking groove (132) so as to unlock the cocking connecting rod (13).

20. A biopsy device, comprising the cocking-unlocking mechanism of the biopsy device according to any one of claims 11-19.

21. A sampling length adjusting mechanism of a biopsy device, comprising an installation frame, wherein a first sliding block (6), a first cocking spring (7), a second sliding block (10) and a second cocking spring (11) are arranged on the installation frame, the first sliding block (6) is fixedly connected to a rear end of a needle tube (2), a front end of the first cocking spring (7) abuts against the first sliding block (6), a rear end of the first cocking spring (7) abuts against the installation frame, the first sliding block (6) is provided with a first elastic barb (61), and after moving backwards in place, the first sliding block (6) is hooked and locked, by the first elastic barb (61) with a first locking portion (85) arranged on the installation frame so as to achieve spring-loading; the second sliding block (10) is fixedly connected to a rear end of a stylet (1), a front end of the second cocking spring (11) abuts against the second sliding block (10), a rear end of the second cocking spring (11) abuts against the installation frame, the second sliding block (10) is provided with a second elastic barb (101), and after moving backwards in place, the second sliding block (10) is hooked and locked, by the second elastic barb (101) with a second locking portion (88) arranged on the installation frame so as to achieve spring-loading; wherein the first sliding block (6) and the second sliding block (10) are in front-back sliding connection to the installation frame and are arranged front and back; and the sampling length adjusting mechanism further comprises a cocking connecting rod (13) for releasing locking and positioning of the first sliding block (6) and/or the second sliding block (10), and a sampling length adjusting component (15), the sampling length adjusting component (15) is provided with a hook boss (151), the sampling length adjusting component (15) can move and at least has a locking state and an unlocking state, when the sampling length adjusting component is in the locking state, the hook boss (151) is arranged on a front-back movement path of the first elastic barb (61) on the first sliding block (6), and the first elastic barb (61) can be hooked with the hook boss (151) for positioning so as to achieve spring-loaded locking, and when the first sliding block (6) is locked at the position, only part of a sampling channel of the stylet (1) can be exposed out of the needle tube (2) so as to implement incomplete-length sampling; and when the sampling length adjusting component is in the unlocking state, the hook boss (151) leaves the front-back movement path of the first elastic barb (61) on the first sliding block (6), and the first elastic barb (61) can freely pass without obstruction.

22. The sampling length adjusting mechanism of the biopsy device according to claim 21, **characterized in that** the cocking connecting rod (13) is provided with a first cocking portion (133) and a second cocking portion (136), the first cocking portion (133) and the second cocking portion (136) are both in a slope shape, and correspondingly, the first elastic barb (61) and the second elastic barb (101) are provided with slopes matched with the first cocking portion (133) and the second cocking portion (136) respectively; when the cocking connecting rod (13) moves front and back for cocking, the second cocking portion (136) firstly thrusts the second elastic barb (101) on the second sliding block (10) to be elastically contracted and deformed and be separated from the second locking portion (88), accordingly, the second sliding block (10) connected to the stylet (1) is unlocked and cocked, then, the first cocking portion (133) thrusts the first elastic barb (61) of the first sliding block (6) to be elastically contracted and deformed and be separated from the first locking portion (85), and accordingly, the first sliding block (6) connected to the needle tube (2) is unlocked and cocked.

23. The sampling length adjusting mechanism of the biopsy device according to claim 22, **characterized in that** the sampling length adjusting component (15) is hinged to an installation frame, can swing up and down, the sampling length adjusting component (15) is provided with the hook boss (151) and a release portion (152), an elastic member (16) makes the sampling length adjusting component (15) rotate and makes the hook boss (151) get onto a front-back movement path of the first elastic barb (61) on the first sliding block (6); the first elastic barb (61) of the first sliding block (6) moves backwards to be hooked with the hook boss (151) for positioning so as to achieve spring-loaded locking, and when the first sliding block (6) is locked at the position, only part of the sampling channel of the stylet (1) can be exposed out of the needle tube (2); a front end of the cocking connecting rod (13) is provided with a thrust portion (137), and when the cocking connecting rod (13) moves forwards, the thrust portion (137) abuts against the release portion (152) and can promote the sampling length adjusting component (15) to rotate so as to enable the hook boss (151) to retreat from the front-back movement path of the first elastic barb (61).

24. The sampling length adjusting mechanism of the biopsy device according to claim 23, **characterized in that** the thrust portion (137) is located on one side of the first cocking portion (133), a contact surface, making contact with the release portion (152), of the thrust portion (137) is a slope or a cambered surface, and a contact surface, making contact with the thrust portion (137), of the release portion (152) is a slope or a cambered surface.

25. The sampling length adjusting mechanism of the biopsy device according to claim 23, **characterized in that** the thrust portion (137) and the first locking portion (85) are located on a left side and a right side of the first cocking portion (133) respectively, the thrust portion (137) is located on a left side or a right side of the front-back movement path of the first elastic barb (61), and a width of a free end of the first elastic barb (61) is larger than that of the thrust portion (137).

26. The sampling length adjusting mechanism of the biopsy device according to claim 23, **characterized in that** the sampling length adjusting component (15) is in a rod shape extending front and back, a hinge portion (153) is arranged on a left side and a right side of the sampling length adjusting component (15),and the hinge portion (153) inserted in a hinge hole (89) in the installation frame so that the sampling length adjusting component (15) can be hinged to the installation frame; and the elastic member (16) is a torsion spring sleeving the hinge portion (153), one end of the torsion spring abuts against the installation frame, the other end of the torsion spring abuts against a front end of the sampling length adjusting component (15), and the release portion (152) is located at a rear end of the sampling length adjusting component (15).

27. The sampling length adjusting mechanism of the biopsy device according to claim 23, **characterized in that** a front portion of the installation frame is provided with a first slideway (81), the first sliding block (6) and the first cocking spring (7) are arranged in the first slideway (81), a rear portion of the installation frame is provided with a second slideway (83), the second sliding block (10) and the second cocking spring (11) are arranged in the second slideway (83), barrier portions are arranged at a front end of the first slideway (81), a rear end of the second slideway (83) and between the first slideway (81) and the second slideway (83), accordingly, the first sliding block (6) is limited to slide in the first slideway (81), and the second sliding block (10) is limited to slide in the second slideway (83); the first locking portion (85) is a convex lug, a front side of the first locking portion (85) is provided with a slope surface for promoting the first elastic barb (61) on the first sliding block (6) to be elastically contracted and deformed, a rear side of the first locking portion (85) is a clamping surface capable of being clamped and locked to a free end of the first elastic barb (61), the second locking portion (88) is a convex lug, a front side of the second locking portion (88) is provided with a slope surface for promoting the second elastic barb (101) on the second sliding block (10) to be elastically contracted and deformed, and a rear side of the second locking portion (88) is a clamping surface capable of being clamped and locked to a free end of the second elastic barb (101); the first slideway (81) and the second slideway (83) are jointly formed by a first installation frame (8) and a second installation frame (22), the cocking connecting rod (13) slides front and back along a sliding groove formed between the first installation frame (8) and the second installation frame (22), the first cocking portion (133) and the second cocking portion (136) are arranged on one side, close to the first slideway (81) and the second slideway (83), of the cocking connecting rod (13), the first locking portions/the first locking portion (85) are/is arranged on the first installation frame (8) and/or the second installation frame (22), located between the cocking connecting rod (13) and the first slideway (81), the first locking portion (85) and located on two or one side of the first cocking portion (133), the first elastic barb (61) is arranged on one side, close to the cocking connecting rod (13), of the first sliding block (6), a front-back movement path of the first elastic barb (61) passes by the first locking portion (85) and partially coincides with a front-back movement path of the first cocking portion (133), and a width of a free end of the first elastic barb (61)is larger than that of the first cocking portion (133); the second locking portions/the second locking portion (88) are/is located on the first installation frame (8) and/or the second installation frame (22), located between the cocking connecting rod (13) and the second slideway (83), and the second locking portion (88) is located on two or one side of the second cocking portion (136), a second elastic barb (101)is arranged on one side, close to the cocking connecting rod (13), of the second sliding block (10), a front-back movement path of the second elastic barb (101) passes by the second locking portion (88) and partially coincides with a front-back movement path of the second cocking portion (136), and a width of a free end of the second elastic barb (101) is larger than of the first cocking portion (136).

28. The sampling length adjusting mechanism of the biopsy device according to claim 23, **characterized in that** when the first sliding block (6) is in a spring-loaded locking state, the first locking portion (85) and the first cocking portion (133) are located on a front side and a rear side of a free end of the first elastic barb (61) respectively, and when the second sliding block (10) is in a spring-loaded locking state, the second locking portion (88) and the second cocking portion (136) are located on a front side and a rear side of the free end of the second elastic barb (101) respectively.

29. A biopsy device, comprising the sampling length adjusting mechanism of the biopsy device according to any one of claims 21-28, **characterized in that** the installation frame is further provided with a spring-loading cocking mechanism for driving the first sliding block (6) and the second sliding block (10) to be moved backwards, locked and positioned and compressing the first cocking spring (7) and the second cocking spring (11) for potential storage; and the spring-loading cocking mechanism comprises a first spring-loading button (14) and a second spring-loading button (19), the first spring-loading button (14) is connected to the first sliding block (6) and can drive the first sliding block (6) to move backwards, and the second spring-loading button (19) is connected to the second sliding block (10) and can drive the second sliding block (10) to move backwards.

30. A spring-loading cocking method for the biopsy device according to claim 29, comprising the following steps:
1) spring-loading,
wherein, the first spring-loading button (14) and the second spring-loading button (19) are respectively pressed; the first sliding block (6) is driven by the first spring-loading button (14) to move backwards and compress the first cocking spring (7) until the first elastic barb (61) on the first sliding block (6) is locked with the hook boss (151) on the sampling length adjusting component (15), and the first sliding block (6) and the needle tube (2) finish spring-loading; and the second sliding block (10) is driven by the second spring-loading button (19) to move backwards and compress the second cocking spring (11) until the second elastic barb (101) on the second sliding block (10) is locked, and the second sliding block (10) and the stylet (1) finish spring-loading; and
2) cocking,
wherein the rear cocking button (131) is pressed or the side cocking button (17) is forwards pushed to drive the cocking connecting rod (13) to move forwards;
firstly, the cocking connecting rod (13) promotes, by a second cocking portion (136), the second elastic barb (101) to be elastically deformed and separated from a second locking portion (88) so as to unlock the second sliding block (10), and the second sliding block (10) is pushed by the second cocking spring (11) to move forwards to finish unlocking and cocking of the second sliding block (10) and the stylet (1); at the same time, the first cocking portion (133) and the thrust portion (137) of the cocking connecting rod (13) do not make contact with the first elastic barb (61) on the first sliding block (6) to maintain the locking state of the first sliding block (6); accordingly, a part of the sampling channel of the stylet (1) is exposed out of the front end of the needle tube (2);
then, along with continuous forward movement of the cocking connecting rod (13), the thrust portion (137) at the front end of the cocking connecting rod (13) abuts against a release portion (152) and can promote the sampling length adjusting component (15) to rotate so as to enable the hook boss (151) to retreat from the front-back movement path of the first elastic barb (61) so that clamping and locking, by the hook boss (151), on the first elastic barb (61) can be released, the first sliding block (6) is pushed by the first cocking spring (7) to move forwards to finish unlocking and cocking of the first sliding block (6) and the needle tube (2), and the needle tube (2) moves forwards to accommodate the sampling channel of the stylet (1) in the front end; and
finally, the rear cocking button (131) and the side cocking button (17) are released, the cocking connecting rod (13) is pushed by the reset spring (18) to move backwards and reset, and the sampling length adjusting component (15) resets under the action of the elastic member (16).
